# FASCICULE DE BREVET EUROPEEN

(11) **EP 0 926 956 B1**
(45) Date de publication et mention de la délivrance du brevet: **05.07.2000**
(21) Numéro de dépôt: 97940217.9
(22) Date de dépôt: 11.09.1997
(51) Int. Cl.: A01N 65/00, A61K 7/48, A61K 7/06

(54) **UTILISATION D'UN EXTRAIT D'ALGUES EN TANT QU'AGENT ANTIBACTERIEN ET/OU ANTIFONGIQUE ET COMPOSITION COMPORTANT UN TEL EXTRAIT**
VERWENDUNG EINES ALGENEXTRAKTS ALS BAKTERIZID UND/ODER FUNGIZID UND IHN ENTHALTENDE ZUSAMMENSETZUNG
USE OF ALGAE EXTRACT AS ANTIBACTERIAL AND/OR ANTIFUNGAL AGENT AND COMPOSITION CONTAINING SAME

(30) Priorité: 12.09.1996 FR 9611149
(43) Date de publication de la demande: 07.07.1999
(73) Titulaire: ALGUES ET MER S.à.r.l., 29242 Ile d'Ouessant (FR)
(72) Inventeur: MOIGNE, Jean-Yves, F-75116 Paris (FR)
(74) Mandataire: Ballot, Paul Denis Jacques
(86) Numéro de dépôt international: FR9701603
(87) Numéro de publication internationale: WO9810656

(56) Documents cités:
- WO-A-84/02652
- BIOLOGICAL ABSTRACTS, vol. 90, Philadelphia, PA, US; abstract no. 312461, XP002032483 & BANDARA ET AL.: "Antimicrobial activity of some marine algae" J NATL SCI COUNC SRI LANKA, vol. 16, no. 2, 1988, pages 209-222,
- BIOLOGICAL ABSTRACTS, vol. 82, Philadelphia, PA, US; abstract no. 159831, XP002032484 & CHENIEUX ET AL.: "Seaweeds of the french atlantic coast with anti mitotic compounds" PLANT MED O (SUPPL), 1980, pages 152-162,
- BIOLOGICAL ABSTRACTS, vol. 80, Philadelphia, PA, US; abstract no. 153462, XP002032485 & HENRIQUEZ ET AL.: "Antibiotic properties of marine algae 2. Screening of chilean marine algae for anti microbial activity" BOT MAR, vol. 22, no. 7, 1979, pages 451-454,
- BIOLOGICAL ABSTRACTS, vol. 95, Philadelphia, PA, US; abstract no. 489453, XP002032486 & MAHASNEH ET AL.: "Antibiotic activity of marine algae against multi-antibiotic resistant bacteria" MICROBIOS, vol. 83, no. 334, 1995, pages 23-26,
- MC. CONNELL ET AL.: "Halogen Chemistry of the red Alga Asparagopsis" PHYTOCHEMISTRY, vol. 16, 1977, pages 367-374, XP002032482 cité dans la demande
- DATABASE WPI Derwent Publications Ltd., London, GB; AN 95-011755 XP002032488 & JP 06 298 661 A

## Description

L'invention concerne un extrait d'algues comportant des composés organiques halogénés, et une utilisation de cet extrait en tant qu'agent antibactérien, bactériostatique ou bactéricide, et/ou antifongique, fongistatique ou fongicide.

Certaines algues sont connues pour posséder une activité antibactérienne ou antifongique. Il s'agit notamment des Rhodophycées mais aussi des Chlorophycées et des Phéophycées. Parmi les Rhodophycées, les algues de la famille des Bonnemaisoniacées et, en particulier, les algues de l'espèce Asparagopsis, sont connues pour posséder une activité antibactérienne et antifongique remarquable. A ce sujet, on se rapportera au document intitulé "Algues fixées de la côte atlantique française contenant des substances antibactériennes et antifongiques" (J.F. Biard et coll., Planta medica, Journal of medicinal plant research, 1980, supplément, pages 136-151).

Asparagopsis armata est une algue marine le plus souvent épiphyte originaire d'Australie ou de Nouvelle Zélande. Cette algue est présente dans l'hémisphère nord depuis les années 1920 et on peut la trouver en Angleterre, en Irlande, en France, en Espagne, en Italie et au Maroc où elle forme toutefois des populations très clairsemées et instables à des profondeurs allant jusqu'à dix mètres. C'est l'une des raisons pour lesquelles une exploitation industrielle des composés organiques halogénés que comportent ces algues, prélevées par cueillette dans leur milieu naturel, n'a pas été envisagée.

Une autre raison est donnée dans un article intitulé "Halogen chemistry of the red alga Asparagopsis" (Olivier Mc Connell and William Fenical, Phytochemistry, 1977, vol.16, pages 367-374). Les composés organiques halogénés des algues, qui seraient, selon les auteurs de cet article, à l'origine de leur activité antibactérienne et antifongique, comportent 1 à 4 atomes de carbone et sont très volatils. C'est pourquoi ces auteurs ne proposent qu'une méthode d'extraction complexe de ces composés, mise en oeuvre en laboratoire, avec entraînement des molécules volatiles dans un courant d'air chaud suivie d'une extraction dirigée multi-solvants.

Or, le demandeur a développé une technique de culture par microbouturage décrite dans la demande française déposée le 22 mars 1995 sous le numéro 95.03577. Grâce à cette technique, il est possible d'obtenir rapidement une quantité importante d'algues Asparagopsis exploitable industriellement.

Considérant ce qui précède, un problème que se propose de résoudre l'invention est d'obtenir un extrait d'algues clarifié à partir d'un procédé qui puisse être mis en oeuvre industriellement sur une quantité d'algues importante, et de l'utiliser pour l'obtention d'une composition à activité antibactérienne et/ou antifongique.

Une solution à ce problème consiste à effectuer une clarification par filtration du contenu intracellulaire des algues de manière à obtenir un perméat comportant une fraction moléculaire possédant des molécules ayant un poids moléculaire supérieur à 10.000, ladite fraction étant à l'origine, du moins en partie, de l'activité antibactérienne et/ou antifongique.

En définitive, l'invention a pour objet une utilisation d'un extrait d'algues clarifié pour l'obtention d'une composition à activité antibactérienne et/ou antifongique, ledit extrait étant obtenu selon un procédé comportant des étapes de libération du contenu intracellulaire des cellules des algues et de clarification par filtration dudit contenu intracellulaire en vue d'obtenir un perméat de filtration clarifié, ledit extrait comportant une fraction moléculaire possédant des molécules organiques halogénées ayant un poids moléculaire supérieur à 10.000.

Par ailleurs, un tel extrait peut être utilisé pour l'obtention d'une composition cosmétique et/ou pharmaceutique.

La description qui va suivre ne comporte aucun caractère limitatifs Elle est rédigée relativement à l'exemple de l'algue rouge (Rhodophycée) Asparagopsis armata de la famille des Bonnemaisoniacées. Toutefois, il est bien entendu que l'invention s'étend à toute espèce d'algues comprenant des composés organiques intracellulaires halogénés.

Asparagopsis armata est une algue pluricellulaire dont certaines cellules ou vésicules de ces cellules comportent une forte quantité de composés halogénés, notamment bromés et iodés.

Parmi les composés organiques halogénés d'Armata Asparagopsis, on citera les composés suivants, les poids moléculaires desdits composés étant indiqués entre parenthèses :
- halométhanes : CHBr₃ (253), CH₃I (142), CH₂ClI (176), CHCl₃ (119), CCl₄ (152), CHBrCl₂ (163), CHBr₂Cl (208), CBr₄ (332), CHBr₂I (220) ;
- haloacétones : CH₂Br-CO-CH₂Cl (171), CH₂Br-CO-CH₂Br (216), CHBr₂-CO-CH₂Cl (250), CHBrCl-CO-CH₂Br (250), CHBr₂-CO-CH₂Br (295), CHBr₂-CO-CHBrCl (329), CHBr₂-CO-CHBr₂ (374), CH₂Br-CO-CH₃ (137), CHBr₂-CO-CH₃ (216), CHCl₂-CO-CHBrCl (229), CHBr₂-CO-CHCl₂ (285), CHBrCl-CO-CHBrCl (284), CHCl₂-CO-CHCl₂ (196) ;
- haloisopropanols : CHBr₂-CHOH-CH₂Br (297), CHBr₂-CHOH-CHBr₂ (373) ;
- haloacétates : CH₂I-COO-CH₃ (200), CHBr₂-COO-CH₃ (232), CHBr₂-COO-C₂H₅ (246), CHBrI-COO-C₂H₅ (283) ; et
- acides acryliques halogénés : CBr₂=CH-COOH (230), CBr₂=CH-COO-CH₃ (244), CHBr=CH-COO-C₂H₅ (169), CHBr=CI-COO-C₂H₅ (292).

On notera cependant que d'autres composés organiques halogénés ont été signalés dans des algues de la même famille. Ces composés sont présents, dans lesdites algues, en quantité variable selon l'espèce voire, pour une même espèce, selon les lots récoltés.

Pour l'obtention de l'extrait de l'invention, on a cultivé et récolté Asparagopsis armata selon une méthode du type de celle décrite dans la demande française précitée déposée le 22 mars 1995 sous le numéro 95.03577 et dont le contenu est incorporé aux présentes par citation de référence.

L'algue fraîche récoltée est alors conditionnée dans des emballages étanches et vides d'air.

Ces emballages sont congelés à une température de congélation inférieure à 0°C, en pratique de l'ordre de - 18°C. A cette température, l'eau libre contenue dans les cellules des algues, et dans les vésicules que comportent ces cellules, est cristallisée. Comme la température de congélation est atteinte de manière lente, les cristaux ainsi formés sont volumineux et provoquent un début d'éclatement des cellules de l'algue.

Les étapes précitées de conditionnement et de congélation sont avantageusement réalisées dès que possible, après la récolte. De ce fait, les pertes de composés organo-halogénés volatils sont limitées.

Les algues conditionnées et congelées peuvent être conservées et stockées congelées pendant un temps relativement long, plusieurs années en pratique.

Lorsque l'on souhaite obtenir un extrait selon l'invention, on effectue tout d'abord un broyage des algues congelées afin d'obtenir, par effet de cisaillement dû aux cristaux de glace, un éclatement cellulaire avancé. Ce broyage est arrêté dès que la température atteint 0°C.

Au dessus de 0°C, une maturation des algues dans leur emballage se produit. En effet, des enzymes des algues dégradent les carraghénanes (dépolymérisation) et autres composés gélifiants présents dans les algues et la matière intracellulaire des cellules des algues se liquéfie.

C'est la raison pour laquelle une maturation à basse température, 4°C au maximum, est ensuite pratiquée pendant un temps t, par exemple 200 heures, afin d'obtenir une dégradation enzymatique naturelle de polysaccharides sans perte de l'activité objet de l'invention.

Ensuite, on effectue un broyage extrêmement fin des algues maturées de manière que les cellules et vésicules desdites algues éclatent et que l'on obtienne un broyat dans lequel le contenu intracellulaire et intravésiculaire des algues est libéré.

Cette dernière étape de broyage, ainsi que les étapes ultérieures d'obtention de l'extrait de l'invention, sont réalisées à température dirigée inférieure à 10°C, préférentiellement de l'ordre de 4°C.

Il est préférable d'ajuster la quantité de matière sèche du broyat à une valeur au plus égale à 8 % en poids. En pratique, la quantité de matière sèche dans un tel broyat est de l'ordre de 10 à 11 % en poids. Aussi, on ajoute 30 % d'eau. Le rendement d'extraction du contenu cellulaire est ainsi optimisé.

Une centrifugation du broyat est alors effectuée par exemple sur Jouan KR 4-22 à 4500 g pendant 15 min. On obtient une partie résiduaire et un surnageant. La partie résiduaire, essentiellement constituée d'une phase solide, comporte les parois membranaires des cellules des algues. Le surnageant, qui comporte la matrice intracellulaire et, de ce fait, les composés organiques halogénés, est récupéré.

Ce surnageant, dont le pH est d'environ 5,94, est alors préférentiellement acidifié avec de l'acide citrique à une valeur comprise entre 2 et 5, par exemple 3,35.

Il est ensuite clarifié par filtration. A cet effet, on effectue avantageusement une microfiltration tangentielle dudit surnageant. Le seuil de clarification est de l'ordre de 1,5 µm. Au-dessus de cette valeur, il n'y a pas de clarification. Au-dessous, les composés responsables de l'activité de l'extrait de l'invention sont filtrés et la quasi-totalité de l'activité présente initialement dans le surnageant est récupérée, en définitive, à une valeur de l'ordre de 1,4 µm, qui correspond d'ailleurs à peu près au seuil de clarification.

Avec cette étape de microfiltration, et sans étape préalable de séchage ou de mise en solution dans des solvants et d'évaporation, on obtient, selon l'invention, un extrait d'algues clarifié liquide, limpide, et d'une couleur légèrement jaunâtre.

Des essais ont été effectués avec cet extrait en vue d'évaluer ses propriétés antibactérienne et antifongique sur différentes souches bactériennes, levures ou moisissures. Les résultats de ces essais sont rassemblés dans le tableau suivant, dans lequel on a mesuré, pour chaque souche bactérienne, la réduction de population à 20 ou 32°C et à 5, 30 ou 60 minutes. On notera qu'une réduction de 10⁵ correspond à une réduction dite de 5 log requise par les normes en vigueur.

| | 20°C | 20°C | 20°C | 32°C | 32°C | 32°C |
|---|---|---|---|---|---|---|
| | 5 min. | 30 min. | 60 min. | 5 min. | 30 min. | 60 min. |
| Vibrio anguillarum | > 10⁵ | > 10⁵ | > 10⁵ | > 10⁵ | > 10⁵ | > 10⁵ |
| Pseudomonas aeruginosa | ]10 ; 10³] | ]10³ ; 10⁵] | > 10⁵ | ]10³ ; 10⁵] | > 10⁵ | > 10⁵ |
| Escherichia coli | | ]10 ; 10³] | ]10 ; 10³] | | | > 10⁵ |
| Enterobacter gergoviae | | | ]10 ; 10³] | | | > 10⁵ |
| Staphylococcus aureus | | | ]10³ ; 10⁵] | | | > 10⁵ |
| Candida albicans | | | ]10³ ; 10⁵] | | | > 10⁵ |

Ainsi que cela est montré par ce tableau, l'activité antibactérienne de l'extrait est avérée sur les souches bactériennes Vibrio anguillarum, Pseudomonas aeruginosa, Escherichia coli, Enterobacter gergoviae, Staphylococcus aureus ainsi qu'une activité antifongique sur la souche Candida albicans avec un optimum de temps de contact de 60 minutes au maximum.

Une analyse de l'extrait de l'invention montre qu'il comporte 2,03 % en poids de matière sèche obtenue à 120 °C, 21,2 % en poids de ladite matière sèche, calculés à 550°C, étant formés de matière organique. L'analyse de la composition de cette matière sèche a donné les résultats suivants, exprimés en g/kg de matière sèche :

| | |
|---|---|
| Protides | 68,9 |
| Sucres réducteurs | 53,7 |
| Mannitol | 63,0 |
| Sodium | 256 |
| Potassium | 30,5 |
| Calcium | 12,8 |
| Magnésium | 13,2 |
| Plomb | 0,50 |
| Cadmium | 0,25 |
| Zinc | 38,5 |
| Arsenic | 6,0 |
| Cuivre | 6,0 |
| Mercure | < 0,25 |
| Sélénium | < 2,5 |
| Soufre (SO₄) | 55,1. |

En outre, un dosage des halogènes (iode, chlore, brome) présents dans l'extrait a donné les résultats suivants, toujours exprimés en g/kg de matière sèche :

| | |
|---|---|
| Iode total (inorganique et organique) | 8,4 |
| Chlorures | 426 |
| Brome total (inorganique et organique) | 120,5 |

A noter que les résultats ci-dessus, ainsi que la plupart des résultats donnés ci-après, sont fonction des lots d'algues étudiés. Toutefois, il existe une cohérence entre les résultats obtenus, quels que soient ces lots.

Dans une analyse complémentaire, un échantillon de 33 ml d'extrait de l'invention, extrait dans 4 ml d'hexane, a donné, les résultats suivants, exprimés en mg/l :

| | |
|---|---|
| CHBr₂Cl | 0,36 |
| CHBr₃ | 39,4 |
| CH₂I₂ | 0,12 |
| CHBr₂I | 8,4 |
| CHBrI₂ | 0,4 |
| CHI₃ | 0,3 |

Il apparaît donc que, dans l'extrait de l'invention, le bromoforme CHBr₃ est largement majoritaire, même si le dibromo-iodo-méthane est présent en quantité importante. A un niveau très inférieur, on trouve d'autres micromolécules halogénées.

En vue de contrôler que l'activité antibactérienne et/ou antifongique est bien liée à un ensemble de molécules halogénées de faible poids moléculaire, on a préparé une solution aqueuse de bromoforme à 60 mg/l et comparé son activité à celle d'un perméat de nanofiltration au seuil de 400 Daltons ne renfermant donc que des micromolécules, halogénées notamment. Après avoir effectué des inclusions de 1 ml et 0,1 ml sur milieu PCA pendant 48 heures d'une solution mère renfermant le produit à tester et la souche Pseudomonas aeruginosa à la concentration de 1,9.10⁸, on a obtenu des résultats consignés dans le tableau suivant :

| t | inclusions | Bromoforme 60 mg/l | Perméat 400 D |
|---|---|---|---|
| 48 heures | 1 ml | >> | 0 |
| | 0,1 ml | >> | 2,0.10² |

Dans ce tableau, ainsi que dans les tableaux qui suivent, ">>" signifie que les bactéries se sont développées et sont incomptables.

Il apparaît donc que le bromoforme seul, à la concentration de 1,5 fois celle observée dans l'extrait, n'a pas d'activité à l'encontre de la souche Pseudomonas aeruginosa. Par contre, le perméat de nanofiltration à 400 D est actif contre cette souche le second jour suivant sa fabrication. On peut en déduire que l'activité du perméat à 400 D est due à l'action synergique des micromolécules halogénées, même présentes à l'état des traces.

Par ailleurs, on a fractionné le perméat de microfiltration en lui faisant subir une ultrafiltration (uF) ou une nanofiltration (nF) et en mesurant, pour chaque fraction, à différents temps, l'activité antibactérienne vis à vis de la souche Pseudomonas selon le protocole défini ci-dessus. On a obtenu les résultats suivants :

| | | Extrait | Rét. uF | Perm. uF | Rét. nF | Perm. nF |
|---|---|---|---|---|---|---|
| t=2 jours | 1 ml | 0 | 0 | 0 | 0 | 0 |
| | 0,1 ml | 1,0.10² | 2,0.10² | 1,4.10³ | 3,0.10² | 2,0.10² |
| | s.m. | 2,0.10⁸ | 2,0.10⁸ | 2,0.10⁸ | 2,0.10⁸ | 2,0.10⁸ |
| t=1 mois | 1 ml | 0 | 0 | 5.10¹ | 0 | 0 |
| | 0,1 | 0 | 0 | 1,3.10³ | 0 | 0 |
| | ml | 2,0.10⁸ | 2,0.10⁸ | 2,0.10⁸ | 2,0.10⁸ | 2,0.10⁸ |
| | s.m. | | | | | |
| t=2 mois | 1 ml | 0 | 0 | >> | 0 | >> |
| | 0,1 | 1,0.10⁴ | 0 | >> | 0 | >> |
| | ml | 1,2.10⁸ | 1,2.10⁸ | 1,2.10⁸ | 1,2.10⁸ | 1,2.10⁸ |
| | s.m. | | | | | |

Le seuil de l'ultrafiltration correspond à des molécules de 10.000 D et celui de la nanofiltration, à des molécules de 400 D. De ce qui précède, on constate que les fractions perméats de l'ultrafiltration et de la nanofiltration perdent rapidement leur activité, en moins de 2 mois, alors que les rétentats conservent leur activité. Par ailleurs, des analyses pratiquées sur ces lots ont montré une concentration en iode organique supérieure dans les rétentats. On peut donc en déduire que l'activité de l'extrait serait générée par des macromolécules de poids moléculaire supérieur à 10.000 dont la nature n'est pas clairement identifiée à ce jour. Il est probable que ces molécules soient des complexes protéines-polysaccharides renfermant du brome et de l'iode notamment.

Ces macromolécules joueraient le rôle de matrice de réserve. Une dégradation naturelle, peut-être enzymatique desdites macromolécules, aboutirait à la formation de micromolécules halogénées responsables de l'activité. Cela a été confirmé par des essais complémentaires dans lesquels on a montré que l'extrait vieilli comporte 6 fois plus de bromoforme que l'extrait non vieilli et 70 fois plus de dibromo-iodo-méthane.

Les micromolécules halogénées ont un poids moléculaire inférieur à 400. Leur présence dans les perméats d'ultrafiltration et de nanofiltration explique l'activité attestée de ces fractions pendant un temps court. Ces micromolécules se dégradent en effet par la suite en halogénures, en particulier, iodures et bromures inorganiques.

D'autres essais ont été effectués de manière à mesurer l'influence du pH sur l'activité de l'extrait et sur son vieillissement. L'acidification a été effectuée au cours de l'étape dite d'acidification du procédé d'obtention de l'extrait selon l'invention, par l'acide citrique. Les résultats de ces essais sur la souche Pseudomonas sont contenus dans le tableau suivant :

| | | pH = 3,35 | pH = 4,04 | pH = 5,94 |
|---|---|---|---|---|
| t = 2 jours | 1 ml | 0 | 0 | 0 |
| | 0,1 ml | 0 | 0 | 0 |
| | s.m. | 2,0.10⁸ | 2,0.10⁸ | 2,0.10⁸ |
| t = 1 mois | 1 ml | 0 | 0 | 0 |
| | 0,1 ml | 2,2.10³ | 0 | 3,3.10⁴ |
| | s.m. | 2,4.10⁸ | 2,5.10⁸ | 2,8.10⁸ |
| t = 2 mois | 1 ml | 0 | 0 | 0 |
| | 0,1 ml | 1,6.10⁴ | 2,7.10⁴ | >> |
| | s.m. | 3,2.10⁸ | 2,2.10⁸ | 2,2.10⁸ |
| t = 3 mois | 1 ml | 0 | 0 | 0 |
| | 0,1 ml | 0 | 2,0.10⁴ | >> |
| | s.m. | 3,0.10⁸ | 3,1.10⁸ | 3,1.10⁸ |
| t = 4 mois | 1 ml | 0 | 40 | 0 |
| | 0,1 ml | 3,0.10⁴ | > | >> |
| | s.m. | 3,5.10⁸ | 2,9.10⁸ | 2,9.10⁸ |
| t = 5 mois | 1 ml | 0 | 0 | 1,5.10² |
| | 0,1 ml | 0 | 10⁵ | >> |
| | s.m. | 1,8.10⁸ | 5,0.10⁸ | 5,0.10⁸ |
| t = 13 mois | 1 ml | 0 | | |
| | 0,1 ml | 2.10⁴ | | |
| | s.m. | 3,6.10⁸ | | |

L'activité de l'extrait de l'invention se conserve en milieu acide avec un optimum à pH = 3,35. Ainsi, une acidification à une valeur de pH comprise entre 2 et 5, préférentiellement de l'ordre de 3,35, freinerait la dégradation de la matrice et entraînerait une libération lente des actifs halogénés. En outre, cette acidification stabiliserait les micromolécules halogénées qui s'accumulent dans le milieu et maintiennent l'activité pendant plus de 13 mois.

Selon l'invention, l'extrait est utilisé, dans des compositions cosmétiques et/ou pharmaceutiques, comme bactéricide ou bactériostatique, ou fongicide ou fongistatique. L'extrait est en fait bactéricide ou bactériostatique, fongicide ou fongistatique selon sa concentration dans la composition cosmétique et/ou pharmaceutique et selon les souches bactériennes ou selon les levures ou moisissures auxquelles il s'applique.

Les compositions cosmétiques ci-après, données à titre d'exemples, permettront de mieux apprécier la diversité des formulations cosmétiques dans lesquelles l'extrait de l'invention peut être introduit. Dans ces exemples, les noms commerciaux des produits utilisés sont, le cas échéant, écrits entre parenthèses, les quantités des produits étant exprimées en % en poids.

| **Exemple 1 : Emulsion-gel** | |
|---|---|
| (Eumulgin B1) | 2,5 |
| Glycéryl stéarate (Cutina GMS) | 2,5 |
| Huile minérale (Vaseline) | 4,0 |
| (Cire Lanol CTO) | 2,0 |
| Cyclométhicone (Abil K4) | 1,5 |
| Huile d'amande douce | 1,5 |
| Carbomère (Carbopol 2001) | 0,5 |
| Triéthanolamine | 0,5 |
| Glycérine | 3,0 |
| Extrait algual (CLMO2) | 1,0 |
| Extrait de l'invention | 7,0 |
| (Solubilisant LRI) | 0,5 |
| Parfum | 0,3 |
| Colorant | qs |
| Eau | qsp 100 |

| **Exemple 2 : Lotion-gel** | |
|---|---|
| (Dermol L-45) | 0,5 |
| Extrait algual, propylène glycol, eau (CLMO2) | 1,0 |
| Extrait algual, propylène glycol, eau (FCO2) | 0,5 |
| Sodium PCA, sodium lactate, fructose, collagène | 1,0 |
| Extrait de l'invention | 7,0 |
| PPG-26 butéth-26, huile de castor (Solubilisant LRI) | 0,5 |
| Parfum | 0,3 |
| Colorant | qs |
| Acide citrique | qs |
| Eau | qsp 100 |

| **Exemple 3 : Shampooing** | |
|---|---|
| Sodium lauréth sulfate (Texapon N70) | 5,0 |
| Sodium lauréth sulfate, lauryl polyglucose (Plantaren PS10) | 5,0 |
| Cocoamidopropylbétaïne (Dehyton K) | 4,0 |
| Cocoamide DEA, lauréth-12 (Comperlan LS | 1,0 |
| (Glutamate DOE 120) | 0,5 |
| Lauryl méthyl glucéth-10 hydroxypropyl dimonium chloride .(Glucquat 125) | 0,5 |
| Glycérine | 3,0 |
| Extrait algual, propylène glycol, eau (CLMO2) | 1,0 |
| Extrait de l'invention | 7,0 |
| Glycérides PEG-6 caprilique/caprique (Softigen 767) | 2,0 |
| Parfum | 0,3 |
| Chlorure de sodium | 0,2 |
| Colorant | qs |
| Acide citrique | qs |
| Eau | qsp 100 |

Dans les compositions ci-dessus, on notera que l'Eumulgin B1 est un éther polyéthylène glycol de l'alcool cétéaryl dont la formule générale est R(OCH₂-CH₂)ₙOH où R représente un mélange de groupes alkyles dérivés de l'alcool céthyl et stéaryl et n a une valeur moyenne de 12 ; que la cire Lanol CTO est formée, d'une part, d'un mélange d'alcool gras constitué principalement d'alcool céthyl et stéaryl et, d'autre part, d'un éther polyéthylène glycol de l'alcool cétéaryl dont la formule générale est R(OCH₂CH₂)ₙOH où R représente un mélange de groupes alkyles dérivé de l'alcool céthyl et stéaryl et n a une valeur moyenne de 33 ; que le solubilisant LRI est un polyoxypropylène, polyoxéthylène éther de l'alcool butyle de formule générale C₄H₃(OCH₃CHCH₂)ₓ(OCH₃CH₂)_{y}OH où x et y ont une valeur moyenne de 26 ; que le Dermol L-45 est un ester de l'acide lactique possédant un éther polyéthylène glycol de glycérine, contenant une moyenne de 7 moles d'oxyde d'éthylène ; que le Déhyton K est un zwitérion de formule RCONH(CH₂)₃N⁺(CH₃)₂CH₂COO⁻ où RCO représente la fonction acide gras dérivée de l'huile de coco ; que le Comperlan LS est un mélange d'éthanolamides d'acides de noix de coco de formule générale RCON(CH₂CH₂OH)₂ où RC représente la fonction acide gras dérivée de l'huile de coco et d'un éther polyéthylène glycol du lauryl alcool de formule CH₃(CH₂)₁₀CH(OCH₂CH₂)ₙOH ; que le Glucamate DOE120 est un éther polyéthylène glycol du diester d'acide oléique et du méthyle glucose avec en moyenne 120 moles d'oxyde d'éthylène ; que le Glucquat 125 est un sel d'ammonium quaternaire provenant de la réaction entre un méthyle gluceth-10 et un diméthyl dodécylammonium époxide ; et que le Softigen 767 est un glycéride éthoxylé de formule générale RCOOCH₂CHOHCH₂(OCH₂CH₂)ₙOH où RCO est un mélange de radicaux caprylic et capric et n a une valeur moyenne de 6.

En outre, des essais ont été effectués de manière à déterminer l'efficacité de la protection des formules cosmétiques de base par l'extrait à la concentration de 7 % vis à vis du développement dans le temps des germes aérobies mésophiles ou par des levures ou moisissures, en l'absence de tout autre conservateur. Ces essais mesurent le nombre de germes présents à différents temps en jours à température ambiante ou à 45°C. Les résultats obtenus sont rassemblés dans le tableau suivant.

| | | J=0 | J=15 | J=30 |
|---|---|---|---|---|
| | | T=20°C | T=20°C | T=45°C |
| Emulsion-gel | germes aérobies mésophiles | 70 | 10 | <10 |
| | levures moisissures | <10 | <10 | <10 |
| | | <10 | <10 | <10 |
| Lotion-gel | germes aérobies mésophiles | <10 | 10 | <10 |
| | levures moisissures | <10 | <10 | <10 |
| | | <10 | <10 | <10 |
| Shampoing | germes aérobies mésophiles | <10 | <10 | <10 |
| | levures moisissures | <10 | <10 | <10 |
| | | <10 | <10 | <10 |

Ces résultats montrent l'effet bactériostatique et fongistatique de l'extrait de l'invention inclus dans les compositions cosmétiques en exemple et montrent que cet effet se conserve dans le temps, la stabilisation de l'extrait étant suffisante (1 mois à 45°C correspond à 1 an de vieillissement naturel). A noter que, sans l'extrait, le nombre de colonies aurait été de l'ordre de 60.000 voire 500.000 selon les germes.

Dans les exemples qui viennent d'être décrits, l'extrait selon l'invention était utilisé en tant qu'ingrédient technique d'une composition cosmétique afin de protéger cette-dernière contre des risques de contaminations microbiennes et de limiter, voire même supprimer, l'utilisation de conservateurs chimiques.

Cependant, d'autres formes d'utilisations de l'extrait selon l'invention peuvent être envisagées. Ainsi, l'extrait d'algues selon l'invention peut être incorporé, sous forme de concentré, dans une composition cosmétique et/ou pharmaceutique non seulement pour protéger cette composition contre les risques de contaminations microbiennes, mais aussi en tant que produit actif. En effet, selon sa concentration dans la composition cosmétique, l'extrait selon l'invention peut agir, de manière sélective, contre certaines bactéries ou certains champignons responsables de phénomènes désagréables tels que l'apparition de pellicules sur le cuir chevelu ou d'acné sur les peaux jeunes par exemple.

Des essais ont été effectués avec un concentré de l'extrait d'algues clarifié selon l'invention sur différentes souches bactériennes ou levures afin d'évaluer son activité fongicide et bactéricide. Lors de ces essais, une souche bactérienne ou levure a été mise en contact avec une solution, contenant le concentré de l'extrait, pendant précisément 30 minutes et 60 minutes. Après ce contact, des inclusions de 1 ml et 0,1 ml d'une solution mère renfermant le concentré à tester et la souche microbienne ont été effectuées sur milieu PCA. Les résultats de ces essais sont rassemblés dans le tableau ci-dessous, dans lequel on a mesuré, pour chaque souche microbienne, la population après un temps de contact de 30 minutes et de 60 minutes et pour une concentration du concentré de l'extrait variant d'un facteur 10.

| | Temps de contact | solution mère | 0,1 ml | 1 ml |
|---|---|---|---|---|
| Pityrosporum ovale | 30 min | 8,8.10⁶/ml | 2,8.10⁴/ml | 0 |
| | 60 min | 8,8.10⁶/ml | 2,6.10⁴/ml | 0 |
| Staphylococcus aureus | 30 min | 1,9.10⁸/ml | 2.10³/ml | 0 |
| | 60 min | 1,9.10⁸/ml | 2.10³/ml | 0 |
| Staphylococcus epidermidis | 30 min | 2,5.10⁸/ml | | 0 |

La levure Pityrosporum ovale est l'une des principales causes microbiennes d'apparition et de développement de pellicules sur le cuir chevelu.

On observe un abattement de population parfait (de 6 Log) dans l'inclusion de 1 ml et ce quel que soit le temps de contact. Cette réduction de population est supérieure à la réduction de 5 Log requise par les normes en vigueur. Par conséquent, dans une inclusion de 1 ml, le concentré de l'extrait selon l'invention présente une activité fongicide à l'égard de la levure pityrosporum ovale.

En revanche, pour une inclusion de 0,1 ml, c'est à dire pour une dilution d'un facteur 10 du concentré de l'extrait d'algues clarifié selon l'invention, l'activité de cet extrait est plus limitée ( la réduction de population étant de 2 Log), et elle est de type fongistatique.

Ce résultat prouve que l'extrait selon l'invention, à certaines concentrations, peut détruire ou inhiber le développement de la levure Pityrosporum ovale. Cet extrait peut donc être utilisé pour son activité fongicide dans des produits capillaires de traitement pour détruire les pellicules et pour son activité fongistatique dans des produits capillaires d'entretien pour prévenir l'invasion par les pellicules.

Les souches bactériennes aérobie Staphylococcus aureus et Staphylococcus epidermidis, associées à la levure Pityrosporum ovale et aux souches bactériennes anaérobie Propionibacterium acnes et Propionibacterium granulosum, sont les principales causes microbiennes responsables des problèmes sur peaux jeunes et notamment des problèmes d'acné.

Pour les souches Staphylococcus aureus et Staphylococcus epidermidis, on observe également un abattement de population parfait (de 8 Log) dans une inclusion de 1 ml à partir d'un temps de contact de 30 minutes. De plus, en ce qui concerne la souche staphylococcus aureus, pour une inclusion de 0,1 ml, c'est à dire pour une dilution d'un facteur 10 du concentré de l'extrait d'algues testé, l'activité fongicide de cet extrait est toujours avérée sur cette souche puisque l'on observe un abattement de population de 5 Log qui correspond au minimum requis par les normes en vigueur.

En ce qui concerne l'étude de l'activité d'un concentré de l'extrait selon l'invention sur la souche bactérienne anaérobie Propionibacterium acnes, les essais ont été effectués selon un protocole différent. En effet, dans ce cas, on fait croître la souche bactérienne en milieu anaérobie, sur un milieu liquide M20 pendant 7 jours à 25°C. La souche est ensuite inoculée en milieu aérobie. La population d'une souche essai ayant été mise en contact avec un concentré de l'extrait de l'invention pendant une durée de 24 heures est ensuite comparée à la population d'une souche témoin n'ayant pas été mise en contact avec ce concentré. Les résultats de cet essai sont rapportés dans le tableau ci-dessous.

| Propinibacterium acnes | Solution mère | Temps de contact 24 heures |
|---|---|---|
| Témoin | 8.10⁸ | 6.10⁵ |
| Essai | 6.10⁸ | 5.10¹ |

Ces résultats prouvent que le concentré de l'extrait de l'invention présente un effet bactéricide à l'égard de la souche Propionibacterium acnes (on observe une réduction de population de 7 Log) lorsque le temps de contact est relativement long, c'est à dire d'au moins 24 heures. Le fait que l'activité bactéricide est constatée après un temps de contact relativement long signifie que, dans ce cas, les actifs halogénés de l'extrait sont libérés de manière progressive. L'effet bactéricide du concentré de l'extrait est donc avéré sur Propionibacterium acnes, après un temps de contact relativement long, mais il est moins prononcé que sur Staphylococcus aureus, Staphylococcus epidermidis et Pityrosporum ovale.

En revanche, l'extrait d'algues selon l'invention ne semble pas avoir développé jusqu'à ce jour d'activité bactéricide sur la cinquième souche bactérienne mise en cause dans l'apparition de l'acné, à savoir la souche bactérienne anaérobie Propionibacterium granulosum.

En conséquence, un concentré de l'extrait de l'invention, à certaines concentrations, présente une activité bactéricide et bacteriostatique à l'encontre de quatre souches microbiennes sur cinq habituellement mises en cause dans le domaine des peaux jeunes à problèmes et notamment dans le domaine des problèmes d'acné.

A partir de ces résultats, une utilisation de l'extrait selon l'invention en tant que produit actif dans une composition cosmétique, et même pharmaceutique, peut être envisagée.

Les compositions cosmétiques et pharmaceutiques; ci-après données à titre d'exemples, permettront de mieux apprécier la diversité des formulations dans lesquelles un concentré de l'extrait de l'invention peut être introduit en tant que produit actif. Dans ces exemples, les noms commerciaux des produits utilisés sont, le cas échéant, écrits entre parenthèses, les quantités des produits étant exprimées en % en poids.

| **Exemple 4 : Crème antiacnéique** | | |
|---|---|---|
| | **prévention** | **traitement** |
| Eau | 53,65 | 48,65 |
| Propylène glycol | 15,00 | 15,00 |
| PEG-8(Lutrol E 400) | 5,00 | 5,00 |
| Tetrasodium EDTA (Edeta B poudre) | 0,05 | 0,05 |
| Triéthanolamine | 0,20 | 0,20 |
| Acide stéarique | 3,00 | 3,00 |
| Huile de coco hydrogénée | 2,00 | 2,00 |
| polysorbate 60 | 1,80 | 1,80 |
| Stérate de sorbitan | 1,20 | 1,20 |
| huile de germes de blé pure | 0,50 | 0,50 |
| Stéarate de PEG-100 et glyceryl stéarate | 1,30 | 1,30 |
| Huile de vaseline | 10,50 | 10,50 |
| DL α tocopherol | 0,50 | 0,50 |
| **concentré de l'extrait de l'invention** | **5,00** | **10,00** |

| **Exemple 5 : Fluide antiacnéique** | | |
|---|---|---|
| | **prévention** | **traitement** |
| Eau | 65,95 | 60,95 |
| Tetrasodium EDTA (Edeta B poudre) | 0,05 | 0,05 |
| Acide citrique | 0,05 | 0,05 |
| Gomme xanthane | 0,30 | 0,30 |
| Ceteareth-25 (crémophor A25) | 2,00 | 2,00 |
| Steareth-7 (Lamecrème SA7) | 5,00 | 5,00 |
| Stearate de glycérine SE | 3,00 | 3,00 |
| Myristate d'isopropyle | 11,80 | 11,80 |
| Lanolin (Stellux AI) | 1,00 | 1,00 |
| Squalane (Cosbiol) | 5,00 | 5,00 |
| DL α tocopherol | 0,50 | 0,50 |
| **Concentré de l'extrait de l'invention** | **5,00** | **10,00** |
| Fragance (Acacia 887) | 0,35 | 0,35 |

| **Exemple 6 : Gel antiacnéique** | | |
|---|---|---|
| | **prévention** | **traitement** |
| Eau | 87,20 | 82,20 |
| Carbomère (polmère LL) | 0,40 | 0,40 |
| PEG-8 (Lutrol E400) | 2,00 | 2,00 |
| Propylène glycol | 5,00 | 5,00 |
| Triethanolamine | 0,40 | 0,40 |
| **concentré de l'extrait de l'invention** | **5,00** | **10,00** |

| **Exemple 7 : Gel Tonique antipelliculaire** | | |
|---|---|---|
| | **prévention** | **traitement** |
| Eau | 92,00 | 87,00 |
| Carraghénanes (Carraghénate X2) | 3,00 | 3,00 |
| **concentré de l'extrait de l'invention** | **5,00** | **10,00** |

| **Exemple 8 : Lotion antipelliculaire** | | |
|---|---|---|
| | **prévention** | **traitement** |
| Eau | 57,90 | 52,90 |
| Huile de castor hydrogénée PEG-40 | 0,50 | 0,50 |
| Fragance | 0,30 | 0,30 |
| Ethanol | 30,00 | 30,00 |
| Panthenol | 0,50 | 0,50 |
| **concentré de l'extrait de l'invention** | **5,00** | **10,00** |

| **Exemple 9 : Shampooing antipelliculaire** | | |
|---|---|---|
| | **prévention** | **traitement** |
| Eau | 60,65 | 55,65 |
| sodium lauréth sulfate | 25,00 | 25,00 |
| Linoleamide DEA | 2,00 | 2,00 |
| Collagène hydrolysé de potassium cocoyl | 1,50 | 1,50 |
| cocamidopropylbétaïne | 3,00 | 3,00 |
| Tetrasodium EDTA | 0,20 | 0,20 |
| Acide citrique | 0,15 | 0,15 |
| Chlorure de sodium | 2,50 | 2,50 |
| **concentré de l'extrait de l'invention** | **5,00** | **10,00** |

Dans toutes ces formulations, aucun conservateur n'a été ajouté car le concentré de l'extrait de l'invention est utilisé non seulement en tant que produit actif mais aussi pour son rôle d'auto-protection de la composition contre des contaminations microbiennes.

## Revendications

1. Utilisation d'un extrait d'algues clarifié pour l'obtention d'une composition à activité antibactérienne et/ou antifongique, ledit extrait étant obtenu selon un procédé comportant les étapes de libération du contenu intracellulaire des cellules des algues et de clarification par filtration dudit contenu intracellulaire en vue d'obtenir un perméat de filtration clarifié, ledit extrait comportant une fraction moléculaire possédant des molécules organiques halogénées ayant un poids moléculaire supérieur à 10.000.

2. Utilisation d'une fraction moléculaire d'un extrait d'algues clarifié pour l'obtention d'une composition à activité anti-bactérienne et/ou antifongique, ledit extrait étant obtenu selon un procédé comportant les étapes de libération du contenu intracellulaire des cellules des algues et de clarification par filtration dudit contenu intracellulaire en vue d'obtenir un perméat de filtration clarifié, les molécules de ladite fraction ayant un poids moléculaire supérieur à 10.000, des molécules de ladite fraction étant des molécules organiques halogénées.

3. Utilisation selon l'une des revendications 1 ou 2, caratérisée en ce que la filtration est effectuée à une valeur proche du seuil de clarification.

4. Utilisation selon la revendication 3, caractérisée en ce que la filtration est une microfiltration effectuée à une valeur de l'ordre de 1,4 µm et en ce que la valeur du seuil de clarification est de l'ordre de 1,5 µm.

5. Utilisation selon l'une des revendications précédentes, caractérisée en ce que l'extrait est obtenu selon un procédé comportant en outre une étape de congélation des algues après leur récolte à une température de congélation à laquelle l'eau libre contenue dans les cellules de l'algue est cristallisée, la température de congélation étant atteinte de manière lente, un début d'éclatement des cellules de l'algue étant alors provoqué.

6. Utilisation selon la revendication 5, caractérisée en ce que l'extrait est obtenu par un procédé comportant en outre l'étape suivante de broyage des algues congelées de manière à obtenir un broyât dans lequel les cellules desdites algues sont éclatées.

7. Utilisation selon l'une des revendications précédentes, caractérisée en ce que l'extrait est obtenu selon un procédé comportant en outre une étape de dégradation des composés gélifiants des algues en réalisant une maturation des algues à une température supérieure à 0°C pendant un temps t en heures, par exemple 200 heures.

8. Utilisation selon l'une des revendications 6 et 7, caractérisée en ce que l'extrait est obtenu par un procédé comportant en outre une étape d'ajustement de la quantité de matière sèche dans le broyât à une valeur au plus égale à 8 % en poids.

9. Utilisation selon l'une des revendications 6 à 8, caractérisée en ce que l'extrait est obtenu selon un procédé comportant en outre une étape de centrifugation du broyât en vue d'obtenir un surnageant comportant l'activité antibactérienne et antifongique.

10. Utilisation selon l'une des revendications précédentes, caractérisée en ce que l'extrait est obtenu selon un procédé comportant en outre une étape d'acidification à une valeur de pH comprise entre 2 et 5, préférentiellement de l'ordre de 3,35.

11. Utilisation selon l'une des revendications précédentes, caractérisée en ce que les algues sont des Bonnemaisoniacées, en particulier, Asparagopsis armata.

12. Utilisation selon l'une des revendications précédentes pour l'obtention d'une composition cosmétique et/ou pharmaceutique.

13. Utilisation selon l'une des revendications 1 à 11, caractérisée en ce que l'extrait est en outre incorporé, sous forme de concentré, dans une composition cosmétique ou pharmaceutique en tant que produit actif.

## Patentansprüche

1. Verwendung eines geklärten Algenextrakts zur Herstellung einer Zusammensetzung mit antibakterieller und/oder antimykotischer Wirkung, wobei der Extrakt nach einem Verfahren erhalten worden ist, das die Stufen der Freisetzung des intrazellulären Inhalts von Algenzellen und der Klärung dieses intrazellulären Inhalts durch Filtration unter Erhalt eines geklärten Filtrationspermeats umfaßt, wobei der Extrakt eine Molekülfraktion umfaßt, die halogenierte organische Moleküle mit einem Molekulargewicht von mehr als 10000 aufweist.

2. Verwendung einer molekularen Fraktion eines geklärten Algenextrakts zur Herstellung einer Zusammensetzung mit antibakterieller und/oder antimykotischer Wirkung, wobei der Extrakt nach einem Verfahren, das die Stufen der Freisetzung des intrazellulären Inhalts von Algenzellen und der Klärung dieses intrazellulären Inhalts durch Filtration unter Erhalt eines geklärten Filtrationspermeats umfaßt, wobei die Moleküle dieser Fraktion ein Molekulargewicht von mehr als 10000 aufweisen und wobei es sich bei den Molekülen dieser Fraktion um halogenierte organische Moleküle handelt.

3. Verwendung nach einem der Ansprüche 1 oder 2, dadurch gekennzeichnet, daß die Filtration bei einem Wert in der Nähe des Klärungsschwellenwerts durchgeführt wird.

4. Verwendung nach Anspruch 3, dadurch gekennzeichnet, daß es sich bei der Filtration um eine Mikrofiltration handelt, die bei einem Wert in der Größenordnung von 1,4 um durchgeführt wird, und daß der Klärungsschwellenwert in der Größenordnung von 1,5 µm liegt.

5. Verwendung nach einem der vorstehenden Ansprüche, dadurch gekennzeichnet, daß der Extrakt nach einem Verfahren erhalten worden ist, das ferner eine Stufe des Einfrierens der Algen nach ihrer Ernte bei einer Einfriertemperatur, bei der das in den Algenzellen enthaltene freie Wasser kristallisiert, umfaßt, wobei die Einfriertemperatur auf langsame Weise erreicht wird und somit das Aufbrechen der Algenzellen eingeleitet wird.

6. Verwendung nach Anspruch 5, dadurch gekennzeichnet, daß der Extrakt durch ein Verfahren erhalten wird, das ferner die anschließende Stufe des Zerkleinerns der eingefrorenen Algen umfaßt, so daß man ein zerkleinertes Produkt erhält, in dem die genannten Algenzellen aufgebrochen sind.

7. Verwendung nach einem der vorstehenden Ansprüche, dadurch gekennzeichnet, daß der Extrakt nach einem Verfahren erhalten worden ist, das ferner eine Stufe des Abbaus der gelbildenden Verbindungen der Algen umfaßt, indem man eine Reifung der Algen bei einer Temperatur über 0°C für eine Zeitspanne von t Stunden, beispielsweise 200 Stunden, vornimmt.

8. Verwendung nach einem der Ansprüche 6 und 7, dadurch gekennzeichnet, daß der Extrakt durch ein Verfahren erhalten worden ist, das ferner eine Stufe der Einstellung der Trockenmasse im zerkleinerten Produkt auf einen Wert von höchstens 8 Gew.-% umfaßt.

9. Verwendung nach einem der Ansprüche 6 bis 8, dadurch gekennzeichnet, daß der Extrakt nach einem Verfahren erhalten worden ist, das ferner die Stufe der Zentrifugation des zerkleinerten Produkts mit dem Ziel der Bildung eines Überstands, der die antibakterielle und antimykotische Aktivität enthält, umfaßt.

10. Verwendung nach einem der vorstehenden Ansprüche, dadurch gekennzeichnet, daß der Extrakt nach einem Verfahren erhalten worden ist, das ferner eine Stufe des Ansäuerns auf einen pH-Wert von 2 bis 5 und vorzugsweise in der Größenordnung von 3,35 umfaßt.

11. Verwendung nach einem der vorstehenden Ansprüche, dadurch gekennzeichnet, daß es sich bei den Algen um Bonnemaisoniaceen und insbesondere um Asparagopsis armata handelt.

12. Verwendung nach einem der vorstehenden Ansprüche zur Herstellung einer kosmetischen und/oder pharmazeutischen Zusammensetzung.

13. Verwendung nach einem der Ansprüche 1 bis 11, dadurch gekennzeichnet, daß der Extrakt ferner in konzentrierter Form einer kosmetischen oder pharmazeutischen Zusammensetzung als Wirkstoff einverleibt wird.

## Claims

1. Use of a clarified extract of algae for obtaining a composition with antibacterial and/or antifungal activity, the said extract being obtained in accordance with a method including the steps of releasing the intracellular content of the cells of the algae and clarifying the said intracellular content by filtration with a view to obtaining a clarified filtration permeate, the said extract containing a molecular fraction having halogenated organic molecules with a molecular weight greater than 10,000.

2. Use of a molecular fraction of a clarified extract of algae for obtaining a composition with antibacterial and/or antifungal activity, the said extract being obtained in accordance with a method including the steps of releasing the intracellular content of the cells of the algae and clarifying the said intracellular content by filtration with a view to obtaining a clarified filtration permeate, the molecules of the said fraction having a molecular weight greater than 10,000, molecules of the said fraction being halogenated organic molecules.

3. Use according to one of Claims 1 or 2, characterised in that the filtration is carried out at a value close to the clarification threshold.

4. Use according to Claim 3, characterised in that the filtration is a microfiltration carried out at a value of around 1.4 µm and in that the clarification threshold value is around 1.5 µm.

5. Use according to one of the preceding claims, characterised in that the extract is obtained according to a method also including a step of freezing the algae after they are harvested at a freezing temperature at which the free water contained in the cells of the algae is crystallised, the freezing temperature being reached slowly, an incipient bursting of the cells of the algae then being caused.

6. Use according to Claim 5, characterised in that the extract is obtained by a method also including the following step of grinding the frozen algae so as to obtain a grind in which the cells of the said algae are burst.

7. Use according to one of the preceding claims, characterised in that the extract is obtained in accordance with a method also including a step of degradation of the gelling compounds of the algae by effecting a ripening of the algae at a temperature above 0°C for a time t in hours, for example 200 hours.

8. Use according to one of Claims 6 and 7, characterised in that the extract is obtained by a method also including a step of adjusting the quantity of dry material in the grind to a value at the most equal to 8% by weight.

9. Use according to one of Claims 6 to 8, characterised in that the extract is obtained according to a method also including a step of centrifuging the grind with a view to obtaining a supernatant having the antibacterial and antifungal activity.

10. Use according to one of the preceding claims, characterised in that the extract is obtained in accordance with a method also including a step of acidification to a pH value between 2 and 5, preferably around 3.35.

11. Use according to one of the preceding claims, characterised in that the algae are Bonnemaisonia, in particular Asparagopsis armata.

12. Use according to one of the preceding claims for obtaining a cosmetic and/or pharmaceutical composition.

13. Use according to one of Claims 1 to 11, characterised in that the extract is also incorporated, in the form of a concentrate, in a cosmetic or pharmaceutical composition as an active product.
